# EUROPÄISCHE PATENTSCHRIFT

(11) **EP 4 070 768 B1**
(45) Veröffentlichungstag und Bekanntmachung des Hinweises auf die Patenterteilung: **26.02.2025**
(21) Anmeldenummer: 22167163.9
(22) Anmeldetag: 07.04.2022
(51) Int. Cl.: A61F 13/02, A61L 15/30, A61L 15/44, A61F 13/40, B32B 3/26, B32B 7/06, B32B 7/12, A61F 13/00

(54) **PFLASTER ZUR VERWENDUNG IM RAHMEN EINER INTRAMUSKULÄREN INJEKTION, INSBESONDERE EINER IMPFUNG**
PATCH FOR USE IN THE CONTEXT OF INTRAMUSCULAR INJECTION, IN PARTICULAR FOR VACCINATION
PANSEMENT DESTINÉ À ÊTRE UTILISÉ DANS LE CADRE D'UNE INJECTION INTRAMUSCULAIRE, EN PARTICULIER D'UNE VACCINATION

(30) Priorität: 08.04.2021 IT 202100008792
(43) Veröffentlichungstag der Anmeldung: 12.10.2022
(73) Patentinhaber: pfm medical gmbh, 50996 Köln (DE)
(72) Erfinder: Rieck, Monika, 50996 Köln (DE)
(74) Vertreter: Lippert Stachow Patentanwälte Rechtsanwälte Part.mbB

(56) Entgegenhaltungen:
- KR-A- 20200 040 329
- US-A- 5 728 071
- US-A- 6 007 562
- US-B1- 7 129 389

## Beschreibung

Die Erfindung betrifft ein Pflaster zur Verwendung im Rahmen einer intramuskulären Injektion, insbesondere einer Impfung, wie die in den Ansprüchen definiert ist.

Bei einer intramuskulären Injektion wird ein flüssiges Arzneimittel in einen Skelettmuskel injiziert. Die Injektion erfolgt üblicherweise in den Oberarm (Musculus deltoideus), den Oberschenkel (Musculus vastus lateralis) oder das Gesäß (Musculus gluteus medius bzw. minimus). Zur Durchführung der intramuskulären Injektion wird zunächst die beabsichtigte Einstichstelle desinfiziert. Nachfolgend wird mittels einer Kanüle oder Spritze, insbesondere einer Einmalspritze, das flüssige Arzneimittel in den Skelettmuskel injiziert. Nach erfolgter Injektion wird die Einstichstelle mit einem Tupfer abgedeckt, welcher wiederum mit einem Klebeband oder Pflaster fixiert wird, bis die Einstichstelle sich verschlossen hat.

Ausgehend von diesem Stand der Technik liegt der Erfindung die Aufgabe zugrunde, die Durchführung einer intramuskulären Injektion, insbesondere deren Vorbereitung und Nachbehandlung, zu vereinfachen und den dafür benötigten Zeitaufwand zu reduzieren. KR 2020 0040329 A offenbart ein Pflaster mit einer Patientenseite und einer Außenseite, umfassend eine Klebeschicht durch die eine Injektionsnadel eingeführt werden kann und eine Abdeckung, so dass die Abdeckung entfernt werden kann um eine Öffnung freizulegen.

Die Aufgabe wird erfindungsgemäß gelöst durch ein Pflaster zur Verwendung im Rahmen einer intramuskulären Injektion, insbesondere einer Impfung, mit einer Patientenseite und einer Außenseite, umfassend eine Klebeschicht auf der Patientenseite zur Befestigung des Pflasters auf der Haut eines Patienten, eine saugfähige Wundauflage auf der Patientenseite im Bereich einer beabsichtigten Einstichstelle der intramuskulären Injektion, wobei die saugfähige Wundauflage ein Desinfektionsmittel aufgenommen hat, und eine Öffnung auf der Außenseite im Bereich der saugfähigen Wundauflage der Patientenseite für die Injektionsnadel, und wobei das Pflaster die beabsichtigte Einstichstelle vollständig bedeckt und die saugfähige Wundauflage durch die Öffnung sichtbar ist.

Im Rahmen einer intramuskulären Injektion, insbesondere einer Impfung, wird das erfindungsgemäße Pflaster zur Vorbereitung der intramuskulären Injektion mittels der Klebeschicht auf der Patientenseite auf die Haut des Patienten geklebt. Das Pflaster wird so auf die Haut des Patienten geklebt, dass die saugfähige Wundauflage der Patientenseite im Bereich der beabsichtigten Einstichstelle der intramuskulären Injektion angeordnet ist. Dadurch, dass die saugfähige Wundauflage ein Desinfektionsmittel aufgenommen hat, wird automatisch die beabsichtigte Einstichstelle desinfiziert. Die intramuskuläre Injektion kann also nach Aufbringen des Pflasters auf die Haut des Patienten, im Bereich der beabsichtigten Einstichstelle, durch die Öffnung der Außenseite des Pflasters erfolgen. Nach erfolgter intramuskulärer Injektion wird die Injektionsnadel aus der Einstichstelle gezogen. Da das Pflaster bereits die Einstichstelle abdeckt, müssen keine weiteren Schritte zur Nachversorgung der Einstichstelle unternommen werden. Vielmehr kann das Pflaster einfach entfernt werden, wenn sich die Einstichstelle geschlossen hat. Das erfindungsgemäße Pflaster vereinfacht somit die Vorbereitung und Nachbehandlung einer intramuskulären Injektion, so dass diese schneller durchgeführt werden kann. Das erfindungsgemäße Pflaster ist insbesondere für die Durchführung von intramuskulären Injektionen geeignet, da dabei die Injektionsnadel nicht gezielt in ein Blutgefäß eingeführt werden muss, sondern lediglich in einen größeren Muskel. Die genaue Einstichstelle wird daher während der Durchführung der intramuskulären Injektion vollständig von dem Pflaster bedeckt.

In einer erfindungsgemäßen Variante ist die Öffnung auf der Außenseite mit einer Membran verschlossen, welche einen Austritt von Desinfektionsmittel durch die Öffnung verhindert und ein Durchstechen mit der Injektionsnadel ermöglicht. Dadurch wird gewährleistet, dass die beabsichtigte Einstichstelle von dem Desinfektionsmittel desinfiziert wird und dieses nicht durch die Öffnung der Außenseite entweicht. Gleichzeitig kann die Injektionsnadel durch die Membran gestochen werden. Vorzugsweise ist die Membran dabei so ausgebildet, dass diese von der dünnen Injektionsnadel nicht zerstört wird, sondern sich nach der Injektion wieder verschließt. Insbesondere besteht die Membran aus einem Silikon.

Nach einer alternativen Variante der Erfindung ist die Öffnung auf der Außenseite mit einer Abdeckung verschlossen, welche sich zur Durchführung der Injektion entfernen lässt. Die Abdeckung ist dabei zweckmäßigerweise ausgebildet, um einen Austritt von Desinfektionsmittel durch die Öffnung der Außenseite zu verhindern. Zur Durchführung der intramuskulären Injektion kann die Abdeckung der Öffnung der Außenseite entfernt werden.

Gemäß einer vorteilhaften Variante ist die Abdeckung ausgebildet, um die Öffnung auf der Außenseite nach der Injektion wieder zu verschließen. Somit lässt sich die Öffnung der Außenseite des Pflasters nach der intramuskulären Injektion wieder verschließen, wodurch ein Austreten von Desinfektionsmittel oder Wundflüssigkeit bzw. Blut vermieden wird. Ferner wird dadurch die Einstichstelle besser vor äußeren Einflüssen geschützt.

In einer zweckmäßigen Variante der Erfindung ist die Abdeckung als Lasche ausgebildet, welche an einem Randbereich permanent mit der Außenseite des Pflasters verbunden ist. Die Lasche ist somit permanent mit der Außenseite des Pflasters verbunden und kann nicht während der Durchführung der intramuskulären Injektion verlegt, beschädigt, verschmutzt oder dergleichen werden. Wäre die Lasche nicht permanent mit der Außenseite des Pflasters verbunden, müsste diese während der intramuskulären Injektion an einem sicheren und sauberen Ort zwischengelagert werden.

Nach einer erfindungsgemäßen Variante ist die Abdeckung zumindest teilweise selbstklebend. Dadurch kann die Abdeckung beispielsweise wiederholt an der Außenseite des Pflasters befestigt werden, um die Öffnung der Außenseite des Pflasters vor und nach der intramuskulären Injektion zu verschließen. Auch kann die Außenseite des Pflasters in dem Bereich, wo die Abdeckung mit der Außenseite des Pflasters verbunden wird, eine zusätzliche Beschichtung oder dergleichen umfassen, welche das Verbinden und Lösen der Abdeckung mit der Außenseite des Pflasters vereinfacht.

Gemäß einer besonders vorteilhaften Variante der Erfindung ist die saugfähige Wundauflage der Patientenseite im Bereich der Öffnung der Außenseite an der Abdeckung angeordnet. Die saugfähige Wundauflage wird somit im Bereich der Öffnung der Außenseite zusammen mit der Abdeckung entfernt, um die intramuskuläre Injektion durchzuführen. Die Injektionsnadel wird direkt in die Haut des Patienten eingeführt, ohne dass diese die saugfähige Wundauflage durchdringen muss. Dadurch wird verhindert, dass Partikel der Wundauflage an der Injektionsnadel während des Einführens haften bleiben. Nach der intramuskulären Injektion wird die Öffnung vorzugsweise wieder mit der Abdeckung verschlossen und die Einstichstelle wird entsprechend von der saugfähigen Wundauflage bedeckt.

In einer weiteren vorteilhaften Variante der Erfindung umfasst die saugfähige Wundauflage ferner ein betäubendes und/oder schmerzstillendes Mittel, zur Vorbehandlung oder Nachbehandlung der Einstichstelle der intramuskulären Injektion. Dadurch wird die Durchführung der intramuskulären Injektion für die Patienten so schmerzfrei wie möglich durchgeführt. Auch ist es denkbar, dass zur Vorbehandlung und Nachbehandlung unterschiedliche Abdeckungen mit saugfähigen Wundauflagen verwendet werden, welche jeweils unterschiedliche Substanzen aufgenommen haben, beispielsweise ein Desinfektionsmittel und betäubendes Mittel zur Vorbehandlung und ein Desinfektionsmittel und schmerzstillendes Mittel zur Nachbehandlung. Es sind auch andere Kombinationen von Substanzen und Wirkstoffen denkbar.

Nach einer erfindungsgemäßen Variante umschließt die Klebeschicht auf der Patientenseite die Einstichstelle vollständig. So ist beispielsweise auf der Patientenseite am Rand vollumfänglich die Klebeschicht angeordnet, während in der Mitte die saugfähige Wundauflage für die beabsichtigte Einstichstelle angeordnet ist. Die beabsichtigte Einstichstelle ist somit von allen Seiten vor äußeren Einflüssen geschützt und es kann auch kein Desinfektionsmittel der saugfähigen Wundauflage über den Randbereich austreten.

Gemäß einer Variante der Erfindung umfasst das Pflaster auf der Patientenseite eine Trägerschicht, welche vor dem Aufbringen des Pflasters auf die Haut des Patienten entfernt wird. Derartige Trägerschichten sind von Wundpflastern bekannt und schützen die Klebeschicht und Wundauflage auf der Patientenseite vor der Aufbringung des Pflasters auf die Haut des Patienten. Üblicherweise ist diese Trägerschicht zweiteilig ausgebildet, wobei zunächst ein Teil der Trägerschicht entfernt und das Pflaster im Bereich der entfernten Trägerschicht auf die Haut des Patienten aufgeklebt wird. Dabei kann der zweite, nicht entfernte Teil der Trägerschicht zur Handhabung benutzt werden, ohne dass die darunterliegende Klebeschicht berührt wird. Nachfolgend wird der zweite Teil der Trägerschicht entfernt und das Pflaster vollständig auf die Haut des Patienten geklebt.

Vorzugsweise ist die Trägerschicht flüssigkeitsdicht ausgebildet, so dass keine Flüssigkeit an die Klebeschicht oder die saugfähige Wundauflage gelangt und das Desinfektionsmittel der saugfähigen Wundauflage nicht austreten kann.

In einer zweckmäßigen Variante der Erfindung weist das Pflaster eine runde, ovale, quadratische, rechteckige oder mehreckige Form auf.

Die saugfähige Wundauflage ist vorzugsweise mittig auf der Patientenseite des Pflasters angeordnet. Dadurch lässt sich das Pflaster, insbesondere die saugfähige Wundauflage, genauer an der beabsichtigten Einstichstelle platzieren.

Gemäß einer vorteilhaften erfindungsgemäßen Variante ist das Pflaster in einer Umverpackung verpackt, um das Pflaster vor der Verwendung vor äußeren Einflüssen zu schützen. Dies ist beispielsweise auch von Wundpflastern bekannt.

Vorzugweise ist das erfindungsgemäße Pflaster atmungsaktiv ausgebildet, so dass die Haut unter dem Pflaster atmen kann. Dadurch wird ein Verschließen der Einstichstelle beschleunigt.

Zweckmäßigerweise ist die Wundauflage derart ausgebildet, dass diese nicht an der Wunde der Einstichstelle haftet. Diese Ausgestaltung ist beispielsweise auch von Wundpflastern bekannt.

Nachfolgend wird die Erfindung anhand von in den Figuren dargestellten Ausführungsbeispielen näher erläutert. Es zeigen:
- Fig. 1: eine Ansicht der Patientenseite einer ersten Ausführungsform eines erfindungsgemäßen Pflasters,
- Fig. 2: eine Ansicht der Außenseite der ersten Ausführungsform des erfindungsgemäßen Pflasters aus Fig. 1,
- Fig. 3: eine Ansicht der Außenseite einer zweiten Ausführungsform eines erfindungsgemäßen Pflasters,
- Fig. 4: eine Ansicht der Außenseite einer dritten Ausführungsform eines erfindungsgemäßen Pflasters,
- Fig. 5: eine Detailansicht der Abdeckung aus Fig. 4 von der Patientenseite,
- Fig. 6: eine Ansicht der Patientenseite der dritten Ausführungsform des erfindungsgemäßen Pflasters aus Fig. 4,
- Fig. 7: eine Ansicht der Außenseite einer vierten Ausführungsform eines erfindungsgemäßen Pflasters, und
- Fig. 8: eine Detailansicht der Lasche aus Fig. 7 von der Patientenseite.

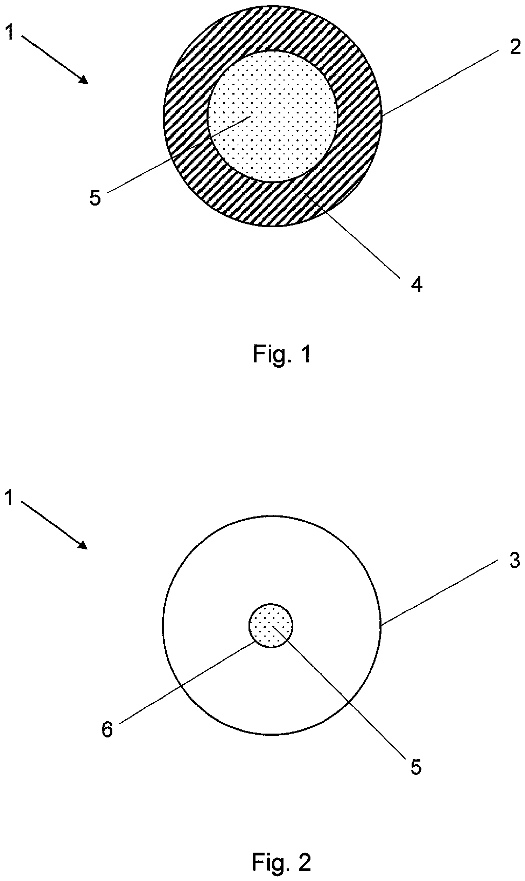

Fig. 1 zeigt eine erste Ausführungsform eines erfindungsgemäßen Pflasters 1 zur Verwendung im Rahmen einer intramuskulären Injektion, insbesondere einer Impfung, mit einer Patientenseite 2 und einer Außenseite 3. Fig. 1 zeigt dabei eine Ansicht der Patientenseite 2 des Pflasters 1.

Die Patientenseite 2 des Pflasters 1 umfasst eine Klebeschicht 4 zur Befestigung des Pflasters 1 auf der Haut eines Patienten und eine saugfähige Wundauflage 5 im Bereich der beabsichtigten Einstichstelle der intramuskulären Injektion. Die Klebeschicht 4 ist entsprechend zu den bekannten Wundpflastern ausgebildet. Wie der Fig. 1 zu entnehmen ist, ist die Klebeschicht 4 am gesamten Umfang des Pflasters 1 angeordnet und umschließt daher die Einstichstelle vollständig. In der Mitte der Patientenseite 2 ist die saugfähige Wundauflage 5 angeordnet, welche von der Klebeschicht 4 umschlossen ist.

Die saugfähige Wundauflage 5 hat erfindungsgemäß ein Desinfektionsmittel aufgenommen, um die Einstichstelle der intramuskulären Injektion zu desinfizieren, sobald das Pflaster 1 auf die Haut des Patienten geklebt wurde. Zweckmäßigerweise hat die saugfähige Wundauflage 5 ein flüssiges Desinfektionsmittel aufgenommen.

Nach einer erfindungsgemäßen Variante umfasst die saugfähige Wundauflage 5 ferner ein betäubendes und/oder schmerzstillendes Mittel zur Vorbehandlung oder Nachbehandlung der Einstichstelle der intramuskulären Injektion. Bei dem betäubenden und/oder schmerzstillenden Mittel handelt es sich vorzugsweise um eine Flüssigkeit, welche von der saugfähigen Wundauflage 5 aufgenommen wurde. Dabei kann es sich insbesondere um kühlende Mittel handeln, welche einen betäubenden und schmerzstillenden Effekt haben.

Fig. 2 zeigt eine Ansicht der Außenseite 3 der ersten Ausführungsform des erfindungsgemäßen Pflasters 1 aus Fig. 1. Die Außenseite 3 des Pflasters 1 weist eine Öffnung 6 im Bereich der saugfähigen Wundauflage der Patientenseite 2 für die Injektionsnadel auf. Durch die Öffnung 6 ist die saugfähige Wundauflage 5 sichtbar. Bei der intramuskulären Injektion wird die Injektionsnadel durch die Öffnung 6 und die saugfähige Wundauflage 5 in die darunterliegende Haut des Patienten eingeführt. Das erfindungsgemäße Pflaster 1 wird dazu so auf der Haut des Patienten platziert, dass sich die Öffnung 6 im Bereich der beabsichtigten Einstichstelle befindet, insbesondere im Bereich des Oberarms (Musculus deltoideus), des Oberschenkels (Musculus vastus lateralis) oder des Gesäßes (Musculus gluteus medius bzw. minimus).

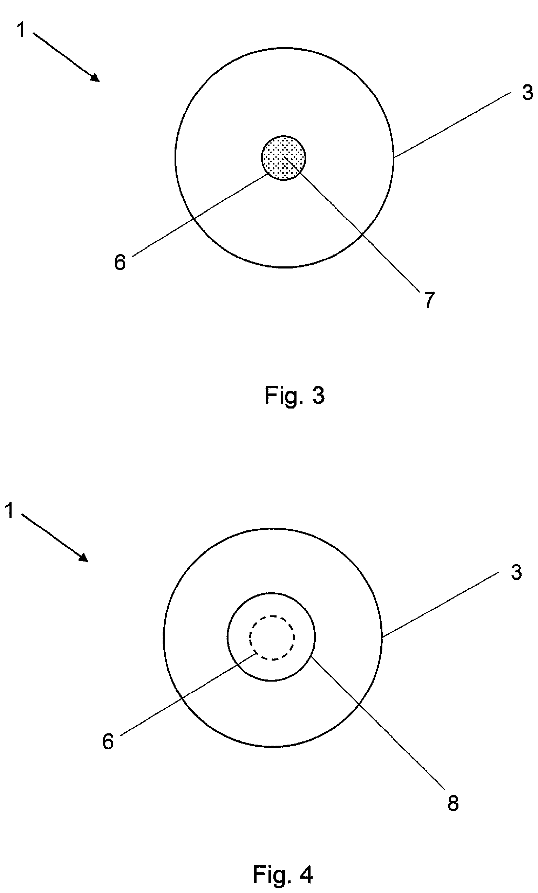

Fig. 3 zeigt eine Ansicht einer Außenseite 3 einer zweiten Ausführungsform eines erfindungsgemäßen Pflasters 1. Die zweite Ausführungsform aus Fig. 3 unterscheidet sich von der ersten Ausführungsform aus den Figuren 1 und 2 dadurch, dass die Öffnung 6 auf der Außenseite 3 mit einer Membran 7 verschlossen ist. Die Membran 7 verhindert einen Austritt von Desinfektionsmittel durch die Öffnung 6 und ermöglicht gleichzeitig ein Durchstechen mit der Injektionsnadel. Aufgrund der Membran 7 ist die saugfähige Wundauflage 5 von der Außenseite 3 nicht sichtbar, außer die Membran 7 ist transparent ausgebildet.

Die Patientenseite 2 der zweiten Ausführungsform aus Fig. 3 ist dabei beispielsweise identisch zur Patientenseite 2 der ersten Ausführungsform, wie in Fig. 1 dargestellt.

Fig. 4 zeigt eine Ansicht einer Außenseite 3 einer dritten Ausführungsform eines erfindungsgemäßen Pflasters 1. Gemäß der dritten Ausführungsform ist die Öffnung 6 auf der Außenseite 3 mit einer Abdeckung 8 verschlossen. Die Abdeckung 8 lässt sich zur Durchführung der intramuskulären Injektion entfernen. Zweckmäßigerweise ist die Abdeckung 8 ausgebildet, um die Öffnung 6 nach der Injektion wieder zu verschließen. Dazu ist die Abdeckung 8 zumindest teilweise selbstklebend ausgebildet, insbesondere in dem Bereich, wo die Abdeckung 8 auf der Außenseite 3 des Pflasters 1 befestigt wird.

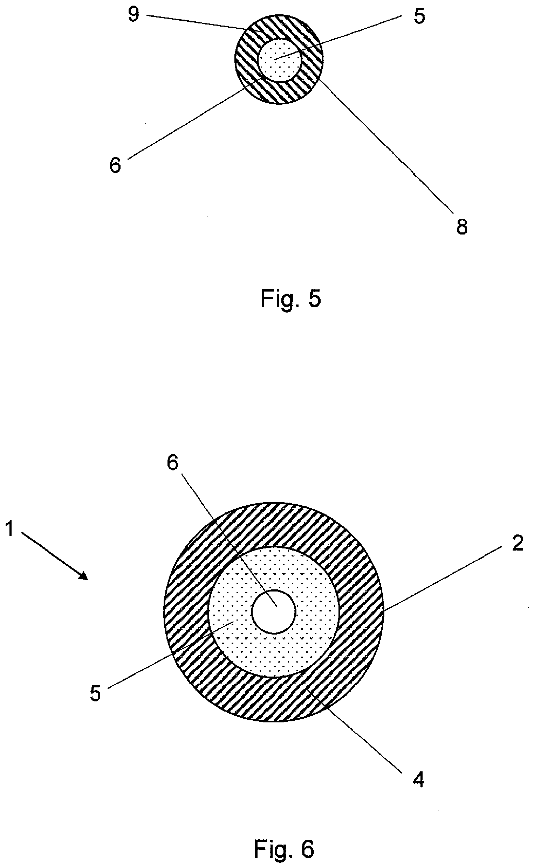

Fig. 5 zeigt eine Detailansicht der Abdeckung 8 von der Patientenseite 2, also der Seite der Abdeckung 8, die auf der Außenseite 3 des Pflasters befestigt wird, um die Öffnung 6 zu verschließen. In dem Bereich, wo die Abdeckung 8 auf der Außenseite 3 des Pflasters 1 aufliegt, weist die Abdeckung 8 eine Klebeschicht 9 auf. In dem Bereich der Öffnung 6 der Außenseite 3 des Pflasters 1 ist die saugfähige Wundauflage 5 vorzugsweise an der Abdeckung 8 befestigt, also innerhalb des Kleberands 9. Dies hat den Vorteil, dass die saugfähige Wundauflage 5 mit der Abdeckung entfernt wird, um die intramuskuläre Injektion durchzuführen und die Einstichstelle sichtbar ist.

Fig. 6 zeigt eine Ansicht der Patientenseite 2 der dritten Ausführungsform des erfindungsgemäßen Pflasters 1 aus Fig. 4. Der Fig. 6 ist zu entnehmen, dass im Bereich der Öffnung 6 die saugfähige Wundauflage 5 an der Abdeckung 8 angeordnet ist und die Öffnung bei entfernter Abdeckung 8 vollständig freigibt, wie in Fig. 6 dargestellt. Außerhalb der Öffnung 6, aber im Bereich um die Öffnung 6, weist die Patientenseite 2 des Pflasters 1 weitere saugfähige Wundauflage 5 auf.

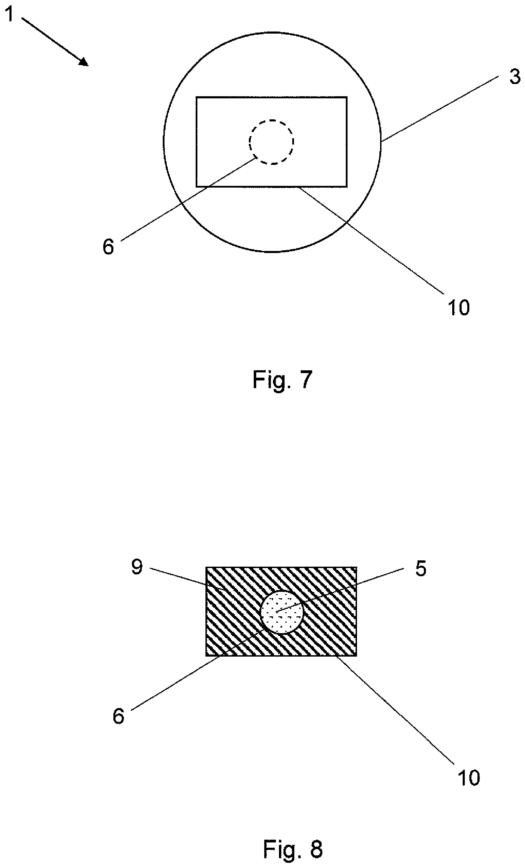

Fig. 7 zeigt eine Ansicht der Außenseite 3 einer vierten Ausführungsform eines erfindungsgemäßen Pflasters 1. Die vierte Ausführungsform aus Fig. 7 unterscheidet sich von der dritten Ausführungsform aus den Figuren 4 bis 6 dadurch, dass die Abdeckung 8 als Lasche 10 ausgebildet ist, welche an einem Randbereich permanent mit der Außenseite 3 des Pflasters 1 verbunden ist. Die Lasche 10 kann zur Durchführung der intramuskulären Injektion aufgeklappt werden, um die Einstichstelle freizugeben, bleibt aber gleichzeitig permanent mit der Außenseite 3 des Pflasters 1 verbunden. Die permanente Verbindung kann beispielsweise durch einen stärkeren Kleber, eine Verschweißung, ein Vernähen oder anderen Verbindungsverfahren hergestellt werden.

Fig. 8 zeigt eine Detailansicht der Lasche 10 aus Fig. 7 von der Patientenseite 2, also der mit der Außenseite 3 des Pflasters 1 verbundenen Seite. Wie bezüglich der dritten Ausführungsform aus den Figuren 4 bis 6 beschrieben, umfasst die Lasche 10 ebenfalls vorzugsweise die saugfähige Wundauflage 5 im Bereich der Öffnung 6 des Pflasters 1. Die der Außenseite 3 des Pflasters 1 zugewandte Oberfläche der Lasche 10 umfasst eine Klebeschicht 9, um die Öffnung 6 des Pflasters 1 wiederholt verschließen zu können. In einer Variante kann die Lasche 10 in einem Randbereich auch einen Abschnitt umfassen, in welchem kein Kleber 9 angeordnet ist, wodurch sich die Handhabbarkeit verbessert, insbesondere wird dadurch das Lösen der Lasche 10 von der Außenseite 3 des Pflasters 1 erleichtert.

Auch wenn die Lasche 10 in Fig. 8 separat dargestellt ist, so ist diese an zumindest einem Randbereich permanent mit der Außenseite 3 des Pflasters 1 verbunden.

Die Patientenseite 2 der vierten Ausführungsform des erfindungsgemäßen Pflasters 1 entspricht beispielsweise der Patientenseite 2 der dritten Ausführungsform des erfindungsgemäßen Pflasters 1 wie in Fig. 6 dargestellt.

### Bezugszeichenliste

- 1: Pflaster
- 2: Patientenseite
- 3: Außenseite
- 4: Klebeschicht (Pflaster)
- 5: saugfähige Wundauflage
- 6: Öffnung für Injektionsnadel
- 7: Membran
- 8: Abdeckung
- 9: Klebeschicht (Abdeckung/Lasche)
- 10: Lasche

## Patentansprüche

1. Pflaster (1) zur Verwendung im Rahmen einer intramuskulären Injektion, insbesondere einer Impfung, mit einer Patientenseite (2) und einer Außenseite (3), umfassend:
eine Klebeschicht (4) auf der Patientenseite (2) zur Befestigung des Pflasters (1) auf der Haut eines Patienten,
eine saugfähige Wundauflage (5) auf der Patientenseite (2) im Bereich einer beabsichtigten Einstichstelle der intramuskulären Injektion, wobei die saugfähige Wundauflage (5) ein Desinfektionsmittel aufgenommen hat, und
eine Öffnung (6) auf der Außenseite (3) im Bereich der saugfähigen Wundauflage (5) der Patientenseite (2) für die Injektionsnadel,
**dadurch gekennzeichnet, dass**
das Pflaster (1) die beabsichtigte Einstichstelle vollständig bedeckt und die saugfähige Wundauflage (5) durch die Öffnung (6) sichtbar ist.

2. Pflaster (1) nach Anspruch 1,
wobei die Öffnung (5) auf der Außenseite (3) mit einer Membran (7) verschlossen ist, welche einen Austritt von Desinfektionsmittel durch die Öffnung (5) verhindert und ein Durchstechen mit der Injektionsnadel ermöglicht.

3. Pflaster (1) nach Anspruch 1,
wobei die Öffnung (5) auf der Außenseite (3) mit einer Abdeckung (8) verschlossen ist, welche sich zur Durchführung der Injektion entfernen lässt.

4. Pflaster (1) nach Anspruch 3,
wobei die Abdeckung (8) ausgebildet ist, die Öffnung auf der Außenseite (3) nach der Injektion wieder zu verschließen.

5. Pflaster (1) nach Anspruch 4,
wobei die Abdeckung (8) als Lasche (10) ausgebildet ist, welche an einem Randbereich permanent mit der Außenseite (3) des Pflasters (1) verbunden ist.

6. Pflaster (1) nach einem der Ansprüche 3 bis 5,
wobei die Abdeckung (8) zumindest teilweise selbstklebend ist.

7. Pflaster (1) nach einem der Ansprüche 1 bis 6,
wobei die saugfähige Wundauflage (5) ferner ein betäubendes und/oder schmerzstillendes Mittel umfasst, zur Vorbehandlung oder Nachbehandlung der Einstichstelle der intramuskulären Injektion.

8. Pflaster (1) nach einem der Ansprüche 1 bis 7,
wobei die Klebeschicht (4) auf der Patientenseite (2) die Einstichstelle vollständig umschließt.

9. Pflaster (1) nach einem der Ansprüche 1 bis 8,
wobei das Pflaster (1) auf der Patientenseite (2) eine Trägerschicht umfasst, welche vor dem Aufbringen des Pflasters (1) auf die Haut des Patienten entfernt wird.

## Claims

1. Plaster (1) for use in the context of an intramuscular injection, particularly a vaccination, having a patient side (2) and an outer side (3), comprising:
an adhesive layer (4) on the patient side (2) for attaching the plaster (1) to the skin of a patient,
an absorbent wound-covering (5) on the patient side (2) in the region of an intended intramuscular injection site, the absorbent wound-covering (5) having received a disinfectant, and
an opening (6) on the outer side (3) in the area of the absorbent wound-covering (5) of the patient side (2) for the injection needle,
**characterized in that**
the plaster (1) totally covers the intended injection site and the absorbent wound-covering (5) is visible through the opening (6).

2. Plaster (1) according to claim 1,
wherein the opening (6) on the outer side (3) is closed with a membrane (7) which prevents disinfectant from escaping through the opening (6) and allows piercing with the injection needle.

3. Plaster (1) according to claim 1,
wherein the opening (6) on the outer side (3) is closed with a cover (8) which can be removed for carrying out the injection.

4. Plaster (1) according to claim 3,
wherein the cover (8) is designed to close the opening on the outer side (3) again after the injection.

5. Plaster (1) according to claim 4,
wherein the cover (8) is formed as a flap (10) which is permanently connected to the outer side (3) of the plaster (1) at an edge region.

6. Plaster (1) according to any one of claims 3 to 5,
wherein the cover (8) is at least partially self-adhesive.

7. Plaster (1) according to any one of claims 1 to 6,
wherein the absorbent wound-covering (5) further comprises an anaesthetic and/or analgesic agent for pre-treatment or post-treatment of the injection site of the intramuscular injection.

8. Plaster (1) according to any one of claims 1 to 7,
wherein the adhesive layer (4) on the patient side (2) completely surrounds the injection site.

9. Plaster (1) according to any one of claims 1 to 8,
wherein the plaster (1) comprises a carrier layer on the patient side (2), which can be removed before the plaster (1) is applied to the patient's skin.

## Revendications

1. Plâtre (1) destiné à être utilisé dans le cadre d'une injection intramusculaire, en particulier d'une vaccination, ayant un côté patient (2) et un côté extérieur (3), comprenant :
une couche adhésive (4) sur le côté patient (2) pour fixer le plâtre (1) à la peau d'un patient,
un pansement absorbant (5) sur le côté patient (2) dans la région d'un site d'injection intramusculaire prévu, le pansement absorbant (5) ayant reçu un désinfectant, et
une ouverture (6) sur le côté extérieur (3) dans la zone du pansement absorbant (5) du côté patient (2) pour l'aiguille d'injection,
**caractérisé en ce que**
le plâtre (1) recouvre entièrement le site d'injection prévu et le pansement absorbant (5) est visible à travers l'ouverture (6).

2. Plâtre (1) selon la revendication 1,
dans lequel l'ouverture (6) sur le côté extérieur (3) est fermée par une membrane (7) qui empêche le désinfectant de s'échapper par l'ouverture (6) et permet le perçage avec l'aiguille d'injection.

3. Plâtre (1) selon la revendication 1,
dans lequel l'ouverture (6) sur le côté extérieur (3) est fermée par un couvercle (8) qui peut être retiré pour effectuer l'injection.

4. Plâtre (1) selon la revendication 3,
dans lequel le couvercle (8) est conçu pour refermer l'ouverture du côté extérieur (3) après l'injection.

5. Plâtre (1) selon la revendication 4,
dans lequel le couvercle (8) est formé d'un rabat (10) qui est relié de façon permanente au côté extérieur (3) du plâtre (1) au niveau d'une zone de bord.

6. Plâtre (1) selon l'une quelconque des revendications 3 à 5,
dans lequel le couvercle (8) est au moins partiellement autocollant.

7. Plâtre (1) selon l'une quelconque des revendications 1 à 6,
dans lequel le pansement absorbant (5) comprend en outre un agent anesthésique et/ou analgésique pour le prétraitement ou le post-traitement du site d'injection de l'injection intramusculaire.

8. Plâtre (1) selon l'une quelconque des revendications 1 à 7,
dans lequel la couche adhésive (4) du côté patient (2) entoure complètement le site d'injection.

9. Plâtre (1) selon l'une des revendications 1 à 8,
dans lequel le plâtre (1) comprend une couche de support sur le côté patient (2), qui peut être enlevée avant que le plâtre (1) ne soit appliqué sur la peau du patient.
